(19) Europäisches Patentamt
European Patent Office
Office européen des brevets

(11) Veröffentlichungsnummer: **0 272 208 B1**

# (12) EUROPÄISCHE PATENTSCHRIFT

(45) Veröffentlichungstag der Patentschrift: **12.06.91**

(51) Int. Cl.[5]: **C07F 9/547**, A61K 31/675

(21) Anmeldenummer: **87810666.5**

(22) Anmeldetag: **16.11.87**

Die Akte enthält technische Angaben, die nach dem Eingang der Anmeldung eingereicht wurden und die nicht in dieser Patentschrift enthalten sind.

## (54) Aromatisch substituierte Azacyclo-alkylalkandiphosphonsäuren.

(30) Priorität: **21.11.86 CH 4664/86**

(43) Veröffentlichungstag der Anmeldung:
**22.06.88 Patentblatt 88/25**

(45) Bekanntmachung des Hinweises auf die Patenterteilung:
**12.06.91 Patentblatt 91/24**

(84) Benannte Vertragsstaaten:
**AT BE CH DE ES FR GB GR IT LI LU NL SE**

(56) Entgegenhaltungen:
**DE-A- 3 232 997**

**SOVIET INVENTIONS ILLUSTRATED, Sektion CH, Klasse B, Seite 3, Nr. 84-010294/02, Woche 8402, Derwent Publications Ltd, London, GB; & SU-A-1 002 300 (HETEROORG. CPDS. INST.) 07-03-1983**

(73) Patentinhaber: **CIBA-GEIGY AG**
**Klybeckstrasse 141**
**CH-4002 Basel(CH)**

(72) Erfinder: **Jaeggi, Knut A., Dr.**
**General Guisan-Strasse 44**
**CH-4054 Basel(CH)**

**EP 0 272 208 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft aromatisch substituierte Azacycloalkylalkandiphosphonsäuren der Formel

$$R - alk - \overset{\displaystyle PO_3H_2}{\underset{\displaystyle PO_3H_2}{C}} - OH \qquad (I)$$

worin R einen durch einen unsubstituierten oder durch $C_1$-$C_4$-Alkyl, $c_1$-$c_4$-Alkoxy und/oder Halogen der Atomnummer bis und mit 35 mono- oder disubstituierten Phenyl-, Pyridyl- oder Thienylrest R' substituierten Pyrrolidino-, Piperidino-, Piperazino-, 3,5-Methylenpiperidino-, d.h. 3-Aza-bicyclo[$3.1^{1,5}.1^{1,5}$]hept-3-yl- oder Octahydroazocinorest R" bedeutet und alk für geradkettiges $C_2$-$C_5$-Alkylen steht, und ihre Salze, insbesondere pharmazeutisch verwendbaren Salze, Verfahren zur Herstellung der erfindungsgemässen Verbindungen, diese enthaltende pharmazeutische Präparate und ihre Verwendung als Arzneimittelwirkstoffe.

Die genannten aromatischen Substituenten R' von Mono- oder Diazacycloaliphathylresten R" sind mit R" vorzugsweise über ein C-Atom verbunden, können aber auch an das gegebenenfalls vorhandene zusätzliche N-Atom gebunden sein.

Der Rest R" ist mit dem Rest alk über ein Stickstoffatom (N-Atom) verbunden.

Reste R sind beispielsweise 3-Phenyl- bzw. 3-(p-Chlorphenyl)-pyrrolidino, 4-Phenyl-, 4-(p-Methoxyphenyl)-, 4-(p-Methylphenyl)-, 4-(p-Chlorphenyl)-bzw. 4-(p-Fluorphenyl)- oder 4-(m-Fluorphenyl)-piperidino, 3-Phenyl-3,5-methylen-piperidino, d.h. 1-Phenyl-3-aza-bicyclo[$3.1^{1,5}.1^{1,5}$]hept-3-yl, 4-(p-Chlorphenyl)-3,5-methylen-piperidino, d.h. 6-(p-Chlorphenyl)-3-aza-bicyclo[$3.1^{1,5}.1^{1,5}$]hept-3-yl, wie exo- oder vor allem endo-4-(p-Chlor-phenyl)-3,5-methylen-piperidino, d.h. exo- oder vor allem endo-6-(p-Chlorphenyl)-3-aza-bicyclo-[$3.1^{1,5}.1^{1,5}$]hept-3-yl, ferner 1-Phenyl- bzw. 1-(p-Chlorphenyl)-3-aza-bicyclo[$3.1^{1,5}.1^{1,5}$]hept-3-yl, 4-Phenylpiperazino und 4-Phenyl-octahydroazocino.

Ferner haben die Allgemeinbegriffe beispielsweise folgende Bedeutungen:

$C_1$-$C_4$-Alkyl ist beispielsweise Methyl, Aethyl, Propyl, Isopropyl oder Butyl, ferner Iso-, Sekundär- oder Tertiärbutyl.

$C_1$-$C_4$-Alkoxy ist beispielsweise Methoxy, Aethoxy, Propyloxy, Isopropyloxy oder Butyloxy, ferner Iso-, Sekundär- oder Tertiärbutyloxy.

Halogen ist beispielsweise Halogen der Atomnummer bis und mit 35, wie Chlor, ferner Fluor oder Brom.

alk $C_2$-$C_5$-Alkylen ist beispielsweise, geradkettiges $C_2$-$C_5$-Alkylen, wie Aethylen, 1,3-Propylen, 1,4-Butylen oder 1,5-Pentylen, ferner Methylen, 1,2-Propylen, 1,2- oder 1,3-Butylen oder 1,4-Pentylen.

Salze von Verbindungen der Formel I sind insbesondere deren Salze mit pharmazeutisch verwendbaren Basen, wie nicht-toxische, von Metallen oder Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, Kupfer-, Aluminium- oder Zinksalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di-oder Triniederalkylaminen, z.B. Methyl-, Aethyl-, Dimethyl- oder Diäthylamin, Mono-, Di- oder Tri-(hydroxyniederalyl)-aminen, wie Aethanol-, Diäthanol- oder Triäthanolamin, Tris-(hydroxymethyl)-amino-methan oder 2-Hydroxytertiärbutylamin, oder N-(Hydroxyniederalkyl)-N,N-diniederalkylaminen bzw. N-(Polyhydroxyniederalkyl)-N-niederalkylaminen, wie 2-(Dimethylamino)-äthanol oder D-Glucamin, oder quaternären aliphatischen Ammoniumhydroxiden, z.B. mit Tetrabutylammoniumhydroxid.

Die Verbindungen der Formel I und ihre Salze weisen wertvolle pharmakologische Eigenschaften auf. Insbesondere besitzen sie eine ausgeprägte regulierende Wirkung auf den Calcium-Stoffwechsel von Warmblütern. Insbesondere bewirken sie an der Ratte eine ausgeprägte Hemmung der Knochenresorption, die sich sowohl in der Versuchsanordnung gemäss Acta Endocrinol. 78, 613-24 (1975) anhand des PTH-induzierten Anstieges des Serumcalciumspiegels nach subcutaner Applikation in Dosen von etwa 0,01 bis etwa 1,0 mg/kg, als auch im TPTX (Thyroparathyroidectomised) - Rattenmodell anhand der durch Vitamin $D_3$ ausgelösten experimentellen Hypercalcämie nach Gabe von Dosen von etwa 0,001 bis 1.0 mg s.c. zeigen lässt. Ebenso wird die durch Walker-256-Tumore induzierte Tumorhyperkalzämie nach peroraler Verabreichung von etwa 1,0 bis etwa 100 mg/kg gehemmt. Ferner zeigen sie in der Adjuvansarthritis der Ratte in der Versuchsanordnung nach Newbould, Brit. J. Pharmacology 21, 127 (1963) sowie nach Kaibara et al., J. Exp. Med. 159, 1388-96 (1984) in Dosen von etwa 0,01 bis 1,0 mg/kg s.c. eine deutliche Hemmung auf das Fortschreiten chronisch-arthritischer Prozesse. Sie sind deshalb vorzüglich geeignet als Arzneimittelwirkstoffe für die Behandlung von Erkrankungen, die mit Störungen des Calciumstoffwechsels in Verbindung gebracht werden können, beispielsweise entzündlicher Prozesse in Gelenken, degenerativer Prozesse

im Gelenkknorpel, von Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arthritis, z.B. Morbus Bechterew, der Neuritis, der Bursitis, Periodontitis und der Tendinitis, der Fibrodysplasie, der Osteoarthrose oder der Artereosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, morbus Paget und die Osteodystrophia fibrosa, ebenso durch Tumore ausgelöste osteolytische Prozesse.

Aus der SU-A-1002300 sind bereits Verbindungen der Formel I, worin R Piperidino oder Morpholino und alk Aethylen bedeutet und aus der DE-A-3 232 997 bereits Verbindungen der Formel I, worin R S,S-Dioxymorpholino und alk Alkylen bedeutet, bekannt und als den Calciumstoffwechsel regulierende Arzneimittelwirkstoffe vorgeschlagen worden.

Die Erfindung betrifft vorzugsweise Verbindungen der Formel I, worin R einen 4-R'-Piperidinorest oder 4-R'-3,5-methylen-piperidino-, d.h. 6-R'-3-Aza-bicyclo[$3.1^{1,5}.1^{1,5}$]hept-3-yl-rest, wie 4-endo R'-3,5-methylen-piperidino, d.h. 6-endo-R'-3-Aza-bicyclo[$3.1^{1,5}.1^{1,5}$]hept-3-yl, bedeutet, wobei R' unsubstituiertes oder durch $C_1$-$C_4$-Alkyl, $C_1$-$C_4$-Alkoxy oder Halogen der Atomnummer bis und mit 35 substituiertes Phenyl darstellt, und alk geradkettiges $C_2$-$C_5$-Alkylen der Formel $-(CH2)_n-$, worin n für eine ganze Zahl von 2 bis und mit 5 steht, bedeutet, und ihre Salze, insbesondere pharmazeutisch verwendbaren Salze.

Die Erfindung betrifft in allererster Linie Verbindungen der Formel I, worin R einen 3-R'-Pyrrolidino- oder 3- oder 4-R'-Piperidinorest bedeutet, wobei R' unsubstituiertes oder durch $C_1$-$C_4$-Alkyl, $C_1$-$C_4$-Alkoxy oder Halogen der Atomnummer bis und mit 35 substituiertes Phenyl darstellt, und alk geradkettiges $C_2$-$C_5$-Alkylen der Formel $-(CH_2)_n-$, worin n für eine ganze Zahl von 2 bis und mit 5 steht, bedeutet, und ihre Salze, insbesondere pharmazeutisch verwendbaren Salze.

Die Erfindung betrifft namentlich die in den Beispielen genannten Verbindungen der Formel I und ihre Salze, insbesondere ihre inneren Salze und pharmazeutisch verwendbaren Salze mit Basen.

Die Erfindung betrifft ferner ein auf an sich bekannten Methoden beruhendes Verfahren zur Herstellung vom Verbindungen der Formel I und ihrer Salze. Dieses ist dadurch gekennzeichnet, dass man

a) in einer Verbindung der Formel

$$R - alk - \underset{X_2}{\overset{X_1}{C}} - OH \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder

b) eine Verbindung der Formel

$$H_2N - alk - \underset{PO_3H_2}{\overset{PO_3H_2}{C}} - OH \qquad (III)$$

oder ein Salz davon mit einem reaktiven Ester eines entsprechenden aromatisch substituierten aliphatischen Dialkohols umsetzt oder

c) eine Verbindung der Formel

R - alk - $X_3$ (IV),

worin $X_3$ Carboxy, Carbamyl oder Cyano, insbesondere Carboxy oder Cyano, bedeutet, mit phosphoriger Säure und Phosphortrichlorid umsetzt, das Primärprodukt hydrolysiert und in einem ausgehend von Verbindungen der Formel IV, worin $X_3$ Cyano oder Carbamyl ist, erhaltenen Zwischenprodukt der Formel

$$R - alk - \underset{PO_3H_2}{\overset{PO_3H_2}{C}} - NH_2 \qquad (V)$$

bzw. einem Salz davon die Aminogruppe durch Behandlung mit salpetriger Säure durch Hydroxy ersetzt und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

Gemäss der Verfahrensvariante a) in Phosphono zu überführende funktionell abgewandelte Phosphonogruppen liegen beispielsweise in einer Esterform, insbesondere einer Diesterform der Formel $-P(=O)(OR)_2$ (IIa) vor, worin OR beispielsweise Niederalkoxy oder eine gegebenenfalls durch Niederalkyl, Niederalkoxy, Halogen, Trifluormethyl und/oder Hydroxy substituierte Phenyloxygruppe bedeutet.

Die Ueberführung einer funktionell abgewandelten in die freie Phosphonogruppe erfolgt in üblicher Weise durch Hydrolyse, beispielsweise in Gegenwart einer Mineralsäure, wie Salz- oder Schwefelsäure, oder durch Umsetzung mit Triniederalkyl-halogensilan, z.B. mit Trimethyl-dichlorsilan oder insbesondere Trimethyl-jodsilan oder Trimethyl-bromsilan, vorzugsweise unter Kühlen, z.B. im Temperaturbereich von etwa 0° bis etwa 25° C.

Die Ausgangsstoffe der Formel II können beispielsweise hergestellt werden, indem man eine Verbindung der Formel

R - alk - COOH (IIb)

oder vorzugsweise das Anhydrid oder Säurechlorid derselben mit einem entsprechenden Phosphorigsäuretriester der Formel $P(OR)_3$ (IIc) in Gegenwart eines Triniederalkylamins, z.B. Triäthylamin, zu einer Verbindung der Formel

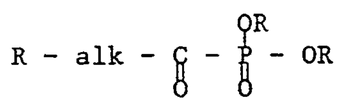

(IId)

und diese mit einem Phosphorigsäurediester der Formel $H-P(=O)(OR)_2$ (IIe) bzw. $P(OH)(OR)_2$ (IIf) in Gegenwart eines Diniederalkylamins, z.B. Diethylamin, oder eines Alkalimetallniederalkanolats, z.B. Natriummethylat, zur entsprechenden Verbindung der Formel

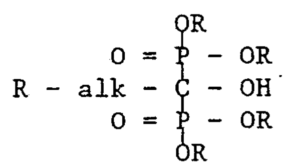

(IIg)

weiterumsetzt.

Ausgangsstoffe der Formel IIb können, soweit sie nicht bekannt sind, beispielsweise hergestellt werden, indem man eine entsprechende Verbindung der Formel

R - H (IIh)

mit einer Verbindung der Formel

Y - alk - COOR (IIi),

worin Y Halogen, wie Brom ist, oder zur Herstellung von Verbindungen IIb, worin alk $C_2$-$C_7$-Alkylen, dessen freie Valenzen von benachbarten C-Atomen ausgehen, z.B. Aethylen, bedeutet, mit einer Verbindung der Formel

alk' - COOR (IIj)

worin alk' einen $C_2$-$C_7$-Alkenylrest bedeutet, umsetzt, jeweils den erhaltenen Ester zur Säure hydrolysiert und diese, z.B. mittels Phosphorpentachlorid, anhydridisiert bzw. chloriert.

Ausgangsstoffe IIh, worin R einen durch R' C-substituierten monocyclischen Azacycloalk(en)ylrest bedeutet, können z.B. hergestellt werden, indem man ein entsprechendes am N-Atom intermediär geschütztes durch eine Oxogruppe substituiertes Azacycloalkan mit einer Verbindung der Formel R'-M (IIk), worin M einen metallischen Rest, z.B. Lithium oder eine Halogenmagnesiumgruppe bedeutet, kondensiert, aus dem erhaltenen Geminal durch R' und Hydroxy substituierten, N-geschützten Azacycloalkan durch Säurebehandlung Wasser abspaltet, die Aminoschutzgruppe entfernt und gewünschtenfalls die Doppel- zur Einfachbindung reduziert.

Ausgangsstoffe IIh, worin R einen durch R' N-substituierten Diazacycloalkylrest bedeutet, können z.B. hergestellt werden, indem man ein entsprechendes $\alpha,\omega$-Dihalogen-azaalkan mit einem Amin der Formel R'-$NH_2$ (III) kondensiert.

Ausgangsstoffe IIh, worin R einen durch R' substituierten 3-Aza-bicyclo[3,1,1]hept-3-ylrest bedeutet, werden z.B. erhalten, indem man ein entsprechendes am N-Atom intermediär geschütztes 2-R'-3,5-dioxo-4-aza-hepta-1,6-dien bzw. 6-R'-2-Br-3,5-dioxo-4-aza-hepta-1,6-dien unter sauren Bedingungen cyclisiert aus dem erhaltenen R'-2,4-Dioxo-3-aza-bicyclo-[3,1,1]heptan-derivat bei N-Schutzgruppe abspaltet und die Oxogruppen sowie das gegebenenfalls vorhandene Bromatom reduktiv durch Wasserstoff ersetzt.

Als Aminschutzgruppen kommen z.B. $\alpha$-Arylniederalkyl, wie Benzyl oder p-Methoxybenzyl, $\alpha,\alpha,\alpha$-Triarylniederalkyl, wie Trityl, oder Triniederalkyl-, wie Trimethylsilyl in Betracht. $\alpha$-Aryl- und $\alpha,\alpha,\alpha$-Triarylniederalkyl kann hydrogenolytisch, Triniederalkylsilyl und $\alpha,\alpha,\alpha$-Triarylniederalkyl hydrolytisch leicht wieder entfernt werden.

Verbindungen IIh, worin R einen gegebenenfalls in 2-, 3- und/oder 4-Stellung niederalkylierten 3-R'-Pyrrolidinorest bedeutet, können besonders elegant ferner hergestellt werden, indem man einen entsprechenden 3-R'-Niederalk-2-en-carbonsäureester mit einem Nitroniederalkan umsetzt, in dem erhaltenen 3-R'-3-(1-Nitroniederalkyl)-niederalkancarbonsäureester die Nitro- zur Aminogruppe hydriert, wobei Ringschluss zum entsprechenden 3-R'-pyrrolidin-5-on erfolgt, und die Oxogruppe reduktiv, z.B. durch Behandeln mit Lithiumaluminiumhydrid oder Diboran, durch Wasserstoff ersetzt.

Gemäss der Verfahrensvariante b) zu verwendende reaktive Mono- oder Diester aromatisch substituierter aliphatischer Dialkohole sind beispielsweise deren Halogen-, wie Chlor-, Brom- oder Jodwasserstoffsäureester, Sulfonsäureester, wie Alkan- oder gegebenenfalls substituierte Benzolsulfonsäureester, wie Methan- oder p-Toluolsulfonate, oder Schwefelsäureester.

Die Umsetzung mit den genannten reaktiven Diestern aromatisch substituierter aliphatischer Dialkohole erfolgt beispielsweise in Gegenwart einer Base, wie eines Alkalimetall- oder Erdalkalimetallhydroxides, z.B. von Natriumhydroxid, oder eines quaternären Ammoniumhydroxides, z.B. von Tetrabutylammoniumhydroxid, vorteilhaft in Gegenwart eines Lösungs-oder Verdünnungsmittels.

Die Ausgangsstoffe der Formel III können beispielsweise hergestellt werden, indem man eine Verbindung der Formel

$H_2N$ - alk - COOH (IIIa)

in üblicher Weise, beispielsweise in Chlorbenzol, mit phosphoriger Säure und Phosphortrichlorid umsetzt und anschliessend hydrolytisch aufarbeitet.

In einer Abwandlung der Verfahrensvariante b) kann man eine Verbindung III auch mit einem reaktiven Mono- oder Diester eines durch R' aromatisch substituierten aliphatischen Alkohols kondensieren und das Primärprodukt der Formel

$$X_4\text{-NH-alk-}\underset{PO_3H_2}{\overset{PO_3H_2}{C}}\text{-OH}$$

worin $X_4$ durch gegebenenfalls reaktionsfähig verestertes Hydroxy substituiertes R'-Aliphatyl bedeutet, cyclisieren.

Die Umsetzung von Verbindungen der Formel IV mit phosphoriger Säure und Phosphortrichlorid gemäss der Verfahrensvariante c) erfolgt in üblicher Weise, wobei die Phophorigsäurekomponente vorzugsweise durch Reaktion überschüssigen Phosphortrichlorides mit wasserhaltiger Phosphorsäure, z.B. mit handelsüblicher etwa 75%-iger bis etwa 95%-iger, vorzugsweise etwa 85%-iger Phosphorsäure, in situ gebildet wird. Die Umsetzung wird vorteilhaft unter Erwärmen, z.B. auf etwa 70° bis etwa 120° C, in einem geeigneten Lösungsmittel, wie Tetrachloräthan, Trichloräthan, Chlorbenzol, Chlortoluol oder Paraffinöl, und unter hydrolytischer Aufarbeitung durchgeführt.

Die Behandlung von Zwischenprodukten der Formel V mit salpetriger Säure erfolgt in üblicher Weise unter Freisetzung derselben in wässriger Lösung aus einem ihrer Salze, z.B. aus Natriumnitrit, durch

Säurebehandlung, z.B. Einwirkung von Salzsäure, wobei intermediär ein entsprechendes, instabiles Diazoniumsalz, z.B. -chlorid, gebildet wird, das unter Einführung der α-Hydroxygruppe Stickstoff abspaltet.

Die Ausgangsstoffe der Formel IV können, soweit sie nicht bekannt sind, beispielsweise hergestellt werden, indem man eine entsprechende Verbindung der Formel

R - H (IIh)

mit einer Verbindung der Formel

Y - alk - $X_3$ (IIi),

worin Y Halogen, wie Brom ist, oder zur Herstellung von Verbindungen der Formel IV, worin alk $C_2$-$C_7$-Alkylen, dessen freie Valenzen von benachbarten C-Atomen ausgehen, z.B. Aethylen bedeutet, mit einer Verbindung der Formel

alk' - $X_3$ (IIf)

worin alk' einen $C_2$-$C_7$-Alkenylenrest bedeutet, umsetzt und gewünschtenfalls jeweils das erhaltene Primärprodukt zur Säure hydrolysiert.

Verfahrensgemäss oder nach einem anderen an sich bekannten Verfahren erhaltene Verbindungen der Formel I können in an sich bekannter Weise in andere Verbindungen der Formel I überführt werden.

So kann man den Rest R' substituieren, beispielsweise durch Umsetzung mit einem üblichen Kernhalogenierungsmittel, z.B. mit Chlor oder Brom in Gegenwart einer Lewissäure, wie Eisen-III-trichlorid, Halogen einführen.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I einschliesslich ihrer inneren Salze der Formel I können durch partielle oder vollständige Neutralisation mit einer der eingangs genannten Basen in Basesalze überführt werden. In analoger Weise kann man auch Säureadditionssalze in die entsprechenden freien Verbindungen oder deren innere Salze überführen.

Umgekehrt kann man erhaltene freie Verbindungen der Formel I durch Behandlung mit einer der eingangs genannten Protonensäuren in Säureadditionssalze überführen.

Erhaltene Salze können in an sich bekannter Weise in die freien Verbindungen umgewandelt werden, z.B. durch Behandeln mit einem sauren Reagens, wie einer Mineralsäure, bzw. einer Base, z.B. Alkalilauge.

Die Verbindungen einschliesslich ihrer Salze können auch in Form ihrer Hydrate erhalten werden oder das zur Krisallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn-und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der Erfindung.

Bei den erfindungsgemässen pharmazeutischen Präparaten, welche Verbindungen der Formel I oder

pharmazeutisch verwendbare Salze davon enthalten, handelt es sich um solche zur enteralen, wie oralen oder rektalen, und parenteralen Verabreichung, welche den pharmakologischen Wirkstoff allein oder zusammen mit einem pharmazeutisch anwendbaren Trägermaterial enthalten.

Die neuen pharmazeutischen Präparate enthalten z.B. von etwa 10 % bis etwa 80 %, vorzugseise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragéee-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. vom Mais-, Weizen-, Reis- oder Kartoffelstärke, Gelatine, Tragaganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulose-präparaten, wie Acetylcellulosephthalat oder Hydroxypropylmethyl-cellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthyleglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls auch Stabilisatoren enthalten.

Die vorliegende Erfindung betrifft ebenfalls die Verwendung der Verbindungen der Formeln I und ihrer Salze, vorzusweise zur Behandlung von auf Störungen des Calciumstoffwechsels zurückzuführende Erkrankungen, z.B. des rheumatischen Formenkreises, und besonders von Osteoporosen.

Dosierungen unter 0,01 mg/kg Körpergewicht beeinflussen die pathologische Verkalkung bzw. die Auflösung von harten Geweben nur unerheblich. Bei Dosierungen von über 100 mg/kg Körpergewicht können langfristig toxische Nebenwirkungen auftreten. Die Verbindungen der Formel I und ihre Salze können sowohl oral als auch in hypertonischer Lösung subkutan, intramuskulär oder intravenös appliziert werden. Die bevorzugten Tagesdosen für diese Anwendungen liegen bei oraler Anwendung im Bereich von etwa 0,1 bis 5 mg/kg, bei subkutaner und intramuskulärer Applikation im Bereich von etwa 0,1 bis 1 mg/kg und bei intravenöser Applikation im Bereich von etwa 0,01 bis 2 mg/kg, beispielsweise von etwa 0,013 bis 0,67 mg/kg.

Die Dosierung der verwendeten Verbindungen ist jedoch variabel und hängt von den jeweiligen

Konditionen wie Art und Schwere der Erkrankung, Dauer der Behandlung und der jeweiligen Verbindung ab. Einzeldosen enthalten beispielsweise von 0,01 bis 10 mg, Dosiseinheitsformen für parenterale, wie intravenöse Applikation z.B. von 0,01 bis 0,1 mg, vorzugsweise 0,02 bis 0,08 mg, orale Dosiseinheitsformen z.B. von 0,2 bis 2,5 mg, vorzugsweise 0,3 bis 1,5 mg pro kg Körpergewicht. Die bevorzugte Einzeldosierung beträgt bei oraler Applikation 10 bis 100 mg und bei intravenöser Applikation 0,5 bis 5 mg und kann bis zu 4-mal täglich verabreicht werden. Die höheren Dosierungen bei oraler Applikation sind infolge der begrenzten Resorption erforderlich. Bei längerdauernden Behandlungen kann nach anfänglich höherer Dosierung normalerweise auf niedrige Dosierungen umgestellt werden, um den gewünschten Effekt aufrechtzuerhalten.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: 26,98 g (0,1 Mol) 3-(4-Phenylpiperidino)propionsäure-hydrochlorid werden mit 13,4 ml 85%-iger Phosphorsäure und 50 ml Chlorbenzol unter Rühren und Rückfluss auf 100° erhitzt. Dann werden bei 100° 27 ml Phosphortrichlorid zugetropft, wobei Gasentwicklung stattfindet. Das Reaktionsgemisch scheidet im Lauf von 30 Minuten eine dicke Masse ab. Man erhitzt noch 3 Stunden auf 100° und dekantiert dann das überstehende Chlorbenzol ab. Die zurückbleibende zähe Masse wird mit 100 ml 9-n Chlorwasserstoffsäure 3 Stunden unter Rühren und Rückfluss zum Sieden erhitzt. Man filtriert heiss unter Kohlezusatz und verdünnt das Filtrat mit Aceton, wobei sich die 3-(4-Phenylpiperidino)-1-hydroxy-propan-1,1-diphosphonsäure kristallin abscheidet; Smp. 243-245° (Zers.) (Ausbeute 57 % d. Th.).

Das als Ausgangsmaterial dienende 3-(4-Phenylpiperidino)propionsäure-hydrochlorid kann folgendermassen hergestellt werden:

25,0 g 4-Phenylpiperidin (0,15 Mol) werden in 50 ml Diäthyläther vorgelegt und unter Rühren allmählich mit 15,1 g Acrylsäureäthylester versetzt. Es bildet sich unter leichtem Anstieg der Temperatur eine klare Lösung. Nach Stehen über Nacht bei Raumtemperatur wird der Aether abdestilliert. Das zurückbleibende Oel stellt den rohen 3-(4-Phenylpiperidino)-propionsäureäthylester dar (Ausbeute ca. 95 %). 39,4 g 3-(4-Phenylpiperidino)-propionsäureäthylester werden mit 600 ml 4-n Chlorwasserstoffsäure 24 Stunden zum Rückfluss erhitzt. Dann wird unter vermindertem Druck völlig eingedampft und der kristalline Rückstand mit Aceton verrieben. Nach Abnutschen, Waschen und Trocknen der Kristalle erhält man das 3-(4-Phenylpiperidino)propionsäure-hydrochlorid, Smp. 216-217°. (Ausbeute 90 % d.Th.)

Beispiel 2: In analoger Weise wie in Beispiel 1 beschrieben kann man ausgehend von jeweils 0,1 Mol
3-(3-Phenylpyrrolidino)propionsäure-hydrochlorid;
3-[4-(p-Methoxyphenyl)piperidino]propionsäure-hydrochlorid;
3-[4-(p-Chlorphenyl)piperidino]propionsäure-hydrochlorid;
3-[4-(p-Fluorphenyl)piperidino]propionsäure-hydrochlorid;
4-(4-Phenylpiperidino)buttersäure-hydrochlorid bzw.
6-(4-Phenylpiperidino)hexansäure-hydrochlorid
auch 3-(3-Phenylpyrrolidino)-1-hydroxy-propan-1,1-diphosphonsäure, Smp: 221° (Zers.);
3-[4-(p-Methoxyphenyl)piperidino]-1-hydroxy-propan-1,1-diphosphonsäure, Smp. 243-245° (Zers.);
3-[4-(p-Chlorphenyl)piperidino]-1-hydroxy-propan-1,1-diphosphonsäure;
3-[4-(p-Fluorphenyl)piperidino]-1-hydroxy-propan-1,1-diphosphonsäure;
4-(4-Phenylpiperidino)-1-hydroxy-butan-1,1-diphosphonsäure, Smp. 230° (Zers.) und
6-(4-Phenylpiperidino)-1-hydroxy-hexan-1,1-diphosphonsäure, Smp. 236-237° (Zers.),
sowie deren Salze, z.B. Dinatriumsalze, herstellen.

Das als Ausgangsmaterial dienende 4-(4-Phenylpiperidino)buttersäure-hydrochlorid und das 6-(4-Phenylpiperidino)hexancarbonsäure-hydrochlorid können wie folgt hergestellt werden:

1,61 g (0,01 Mol) 4-Phenylpiperidin, 2,76 g Kaliumcarbonat und 2,15 g Brombuttersäureäthylester werden in 20 ml 2-Butanon unter Rühren und Rückfluss 24 Stunden erhitzt. Dann wird von den anorganischen Salzen abgenutscht und das Filtrat eingedampft. Der zurückbleibende, rohe 4-(4-Phenylpiperidino)-buttersäureäthylester liefert nach 24-stündigem Kochen mit 40 ml 4-n-Chlorwasserstoffsäure und anschliessendem Eindampfen, Anreiben mit Aceton und Nutschen der Kristalle das 4-(4-Phenylpiperidino)-buttersäure-hydrochlorid, Smp. 217-220° (Zers.).

Analog erhält man, bei Verwendung von 6-Bromhexancarbonsäureäthylester, das 6-(4-Phenylpiperidino)hexancarbonsäure-hydrochlorid, Smp. 197-198°.

Analog erhält man auch ausgehend von
3-Phenylpyrrolidin;
4-(4-Methoxyphenyl)piperidin;
4-(4-Chlorphenyl)piperidin bzw.
4-(4-Fluorphenyl)piperidin;
durch Umsetzung mit Acrylsäureester und anschliessender Hydrolyse mit Salzsäure das

3-(3-Phenylpyrrolidino)propionsäure-hydrochlorid, Smp. 139-140°;
3-[4-(4-Methoxyphenyl)piperidino]-propionsäure-hydrochlorid, Smp. 214° (Zers.);
3-[4-(4-Chlorphenyl)piperidino]-propionsäure-hydrochlorid; und
3-[4-(4-Fluorphenyl)piperidino]-propionsäure-hydrochlorid.

Beispiel 3: In analoger Weise wie in Beispiel 1 beschrieben kann man ausgehend von
4-(p-Methylphenyl)piperidin;
4-(2-Thienyl)piperidin;
4-(3-Thienyl)piperidin;
4-(3-Pyridyl)piperidin;
4-(4-Pyridyl)piperidin;
4-(2-Pyridyl)piperidin;
4-Phenyl-octahydro-azozin bzw. 4-endo-(p-Chlorphenyl)-3,5-methylenpiperidin, d.h. 6-endo-(p-Chlorphenyl)-3-aza-bicyclo[$3.1^{1,5}.1^{1,5}$]heptan, über
3-[4(p-Methylphenyl)piperidino]propionsäure-hydrochlorid;
3-[4-(2-Thienyl)piperidino]propionsäure-hydrochlorid;
3-[4-(3-Thienyl)piperidino]propionsäure-hydrochlorid;
3-[4-(3-Pyridyl)piperidino]propionsäure-hydrochlorid;
3-[4-(4-Pyridyl)piperidino]propionsäure-hydrochlorid;
3-[4-(2-Pyridyl)piperidino]propionsäure-hydrochlorid;
3-(4-Phenyl-octahydro-azocino)propionsäure-hydrochlorid bzw.
3-[4-endo(p-Chlorphenyl)-3,5-methylen-piperidino]propionsäure-hydrochlorid, d.h. 3-[6-endo-(p-Chlorphenyl)-3-aza-bicyclo[$3.1^{1,5}.1^{1,5}$]hept-3-yl]propionsäure-hydrchlorid, Smp. 220° die 3-[4-(p-Methylphenyl)-piperidino]-1-hydroxy-propan-1,1-diphosphonsäure;
3-[4-(2-Thienyl)piperidino]-1-hydroxy-propan-1,1-diphosphonsäure;
3-[4-(3-Thienyl)piperidino]-1-hydroxy-propan-1,1-diphosphonsäure;
3-[4-(3-Pyridyl)piperidino]-1-hydroxy-propan-1,1-diphosphonsäure;
3-[4-(4-Pyridyl)piperidino]-1-hydroxy-propan-1,1-diphosphonsäure;
3-[4-(2-Pyridyl)piperidino]-1-hydroxy-propan-1,1-diphosphonsäure;
3-(4-Phenyl-octahydro-azocino)-1-hydroxy-propan-1,1-diphosphonsäure und
3-[4-endo-(p-Chlorphenyl)-3,5-methylen-piperidino]-1-hydroxy-propan-1,1-diphosphonsäure, d.h. 3-[6-endo-(p-Chlorphenyl)-3-aza-bicyclo-[$3.1^{1,5}.1^{1,5}$]hept-3-yl]-1-hydroxy-propan-1,1-diphosphonsäure, Smp. 224° (Zers.), sowie deren Salze, z.B. Dinatriumsalze, herstellen.

Das als Ausgangsmaterial zu verwendende endo-6-(p-Chlorphenyl)-3-aza-bicyclo[$3.1^{1,5}.1^{1,5}$]heptan(-hydrochlorid) kann folgendermassen hergestellt werden:

a) Zu einer Lösung von 72 g endo-1-Brom-6-(p-chlorphenyl)-3-(p-methoxybenzyl)-3-aza-bicyclo-[$3.1^{1,5}.1^{1,5}$]heptan-2,4-dion und 1,65 g Bisazoisobutyronitril in 1,66 Liter Tetrahydrofuran werden unter einer Stickstoffatmosphäre 49 g Tri-n-butylzinnhydrid gegeben. Die Reaktionslösung wird 1 Stunde zum Rückfluss erwärmt und anschliessend eingeengt. Die so erhaltenen Kristalle werden wie in Beispiel 4d1) beschrieben aufgearbeitet. Man erhält endo-6-(p-Chlorphenyl-3-(4-methoxybenzyl)-3-aza-bicyclo-[$3.1^{1,5}.1^{1,5}$]heptan-2,4-dion in Form weisser Kristalle vom Smp. 156-158°.

b) Zu einer gerührten Suspension von 47,7 g endo-6-(p-Chlorphenyl-3-(p-methoxybenzyl)-3-aza-bicyclo-[$3.1^{1,5}.1^{1,5}$]heptan-2,4-dion in 0,5 Liter Acetonitril wird bei Raumtemperatur eine Lösung von 280 g Cer-(IV)-ammoniumnitrat in 390 ml Wasser zugetropft. Man rührt nach beendeter Zugabe 4 Stunden bei Raumtemperatur nach, destilliert am Wasserstrahlvakuum 200 ml Acetonitril aus dem Reaktionsgemisch ab und verdünnt dann mit 800 ml Wasser. Es wird 1 Stunde im Eisbad gerührt und abgenutscht. Die erhaltenen hellgelben Kristalle werden mit Wasser und Aether gewaschen.
Das Zwichenprodukt wird in 1 Liter Methylenchlorid gelöst, mit 6.8 g n-Propylamin versetzt und über Nacht stehen gelassen. Anschliessend wird filtriert und die hellbraune Lösung auf 70 ml eingeengt. Es wird mit 70 ml Aether verdünnt und abgenutscht. Das so erhaltene grau braune, kristalline endo-6-(p-Chlorphenyl-3-aza-bicyclo[$3.1^{1,5}.1^{1,5}$]heptan-2,4-dion wird mit 50 ml Methylenchlorid-Aether(1:1) gewaschen: Smp. 227-228°.

c) 5,9 g endo-6-(p-Chlorphenyl)-3-aza-bicyclo[$3.1^{1,5}.1^{1,5}$]heptan-2,4-dion werden in 130 ml Toluol mit 24 ml Natriumdihydrobis-(2-methoxyäthoxy)-aluminat-Toluollösung (70 %; FLUKA) umgesetzt und mit 24 ml konzentrierter Natronlauge analog Beispiel 4d2) aufgearbeitet. Man erhält endo-6-(p-Chlorphenyl)-3-aza-bicyclo[$3.1^{1,5}.1^{1,5}$]heptan-hydrochlorid als weisse Kristalle vom Smp. 236-237°.

Beispiel 4: In analoger Weise wie in den Beispielen 1 und 2 beschrieben kann man ausgehend von
4-(m-Fluorphenyl)piperidin;
1-Phenylpiperazin;

3-(p-Chlorphenyl)pyrrolidin;
3-Phenylpyrrolidin;
6-exo-(p-Chlorphenyl)-3-aza-bicyclo[3.1$^{1,5}$.1$^{1,5}$]heptan bzw.
1-Phenyl-3-aza-bicyclo[3.1$^{1,5}$.1$^{1,5}$]heptan über
3-[4-(m-Fluorphenyl)piperidino]propionsäure-hydrochlorid, Smp. 207-208°,
3-(4-Phenylpiperazino)propionsäure-hydrochlorid, Smp. 206-207°;
3-[3-(p-Chlorphenyl)pyrrolidino]-propionsäure-hydrochlorid, Smp. 201;
3-(3-Phenylpyrrolidino]propionsäure-hydrochlorid, Smp. 139-140°;
3-[6-exo-(p-Chlorphenyl)-3-aza-bicyclo[3.1$^{1,5}$.1$^{1,5}$]hept-3-yl]propionsäure-hydrochlorid, Smp. 232° bzw.
3-(1-Phenyl-3-aza-bicyclo[3.1$^{1,5}$.1$^{1,5}$]hept-3-yl)propionsäure-hydrochlorid, Smp. 139-140°, die
3-[4-(m-Fluorphenyl)piperidino]-1-hydroxy-propan-1,1-diphosphonsäure, Smp. 239-240° (Zers.);
3-(4-Phenylpiperazinyl)-1-hydroxy-propan-1,1-diphosphonsäure, Smp. 234° (Zers.) und
3-[3-(4-Chlorphenyl)pyrrolidinyl]-1-hydroxy-propan-l,1-diphosphonsäure, Smp. 219° (Zers.);
3-(3-Phenylpyrrolidino)-1-hydroxy-propan-1,1-diphosphonsäure, Smp. 221° (Zers.);
3-[6-exo-(p-Chlorphenyl)-3-aza-bicyclo[3.1$^{1,5}$.1$^{1,5}$]hept-yl]-1-hydroxypropan-1,1-diphosphonsäure, Smp. 236°, und
3-(1-Phenyl-3-aza-bicyclo[3.1$^{1,5}$.1$^{1,5}$]hept-3-yl)-1-hydroxy-propan-1,1-diphosphonsäure, Smp. 252° (Zers.), sowie deren Salze, z.B. Dinatriumsalze, herstellen.

Das als Ausgangsmaterial zu verwendende exo-6-(p-Chlorphenyl)-3-aza-bicyclo[3.1$^{1,5}$.1$^{1,5}$]heptan(-hydrochlorid) kann folgendermassen hergestellt werden:

a1) Zu einer gerührten Suspension von 100 g Phosphorpentachlorid in 2 Liter Benzol werden unter Rühren 131,6 g N-(4-Methoxybenzyl)-4-chlorzimtsäureamid gegeben. Es wird 30 Minuten bei Raumtemperatur und anschliessend 30 Minuten bei 50° nachgerührt. Nach dem Einengen wird das dunkelbraune Oel in 600 ml Toluol aufgenommen und eingeengt. Der Rückstand wird in 1,2 Liter Tetrachlorkohlenstoff gelöst, mit Diatomeenerde (HYFLO-Super-Cel®) versetzt, abfiltriert, eingedampft und am Vakuum getrocknet.

Das so erhaltene orange, kristalline Zwischenprodukt wird in 1,2 Liter Methylenchlorid gelöst und zu einer Lösung von 430 ml n-Natriumhydrogencarbonatlösung, 6,2 g Tetra-n-butylammoniumbromid und 600 ml Wasser getropft. Nach beendeter Zugabe wird 100 ml n-Natriumhydrogencarbonatlösung zugetropft und 2 Stunden nachgerührt. Die organische Phase wird abgetrennt und die wässrige Phase zweimal mit Methylenchlorid extrahiert. Die vereinigten organischen Phasen werden über Magnesiumsulfat getrocknet, eingeengt, und das Rohprodukt am Hochvakuum getrocknet. Das 4-Aza-2-brom-7-(p-chlorphenyl)-4-(4-methoxybenzyl)-1,6-heptadien-3,5-dion fällt als dunkelbraunes Oel an und wird sofort weiterumgesetzt.

b1) Eine Lösung von 111,8 g 4-Aza-2-brom-7-(4-chlorphenyl)-4-(4-methoxybenzyl)-1,6-heptadien-3,5-dion und 0,6 g 2,6-Di-tert-butyl-p-kresol in 1,4 Liter Xylol wird 2 Stunden am Rückfluss erwärmt. Nach dem Erkalten wird der dunkelbraune Niederschlag von der schwarzen Reaktionslösung getrennt, mit Aether verrührt, abgenutscht und mit Aether gewaschen. Durch fraktionierte Kristallisation aus Acetonitril erhält man zuerst exo-1-Brom-6-(4-chlorphenyl)-3-(4-methoxybenzyl)-3-aza-bicyclo-[3.1.$^{1,5}$.1$^{1,5}$]heptan-2,4-dion als hellgelbe Kristalle mit einem Smp. von 177-180° C und als zweites Produkt die diastereomere Verbindung endo-1-Brom-6-(p-chlorphenyl)-3-(p-methoxybenzyl)-3-azabicyclo[3.1.$^{1,5}$.1$^{1,5}$]-heptan-2,4-dion vom Smp. 142,5-143,5°.

c1) Zu einer gerührten Suspension von 52,1 g exo-1-Brom-6-(p-chlorphenyl)-3-(4-methoxybenzyl)-3-aza-bicyclo[3.1.$^{1,5}$.1$^{1,5}$]heptan-2,4-dion in 2,1 Liter Acetonitril wird bei Raumtemperatur eine Lösung von 249 g Cer(IV)-ammoniumnitrat in 330 ml Wasser zugetropft. Man rührt nach beendeter Zugabe 1 Stunde bei Raumtemperatur nach, erwärmt auf 40°, lässt die Lösung langsam wieder auf Raumtemperatur erkalten und rührt weitere 3 Stunden. Es wird auf ein Drittel des Volumens eingeengt und danach mit 1,6 Liter Wasser verdünnt. Das ausgefallene Produkt wird abgenutscht, mit Wasser, Aether und Essigester gewaschen und am Vakuum getrocknet. Man erhält exo-1-Brom-6-(p-chlorphenyl)-3-aza-bicyclo-[3.1.$^{1,5}$.1$^{1,5}$ ]heptan-2,4-dion als gelbe Kristalle vom Smp. 231-232°.

d1) Zu einer Lösung von 58,5 g exo-1-Brom-6-(p-chlorphenyl)-3-aza-bicyclo[3.1.$^{1,5}$.1$^{1,5}$]heptan-2,4-dion und 1,9 g Bisazoisobutyronitril in 1 Liter Tetrahydrofuran werden unter Stickstoffatmosphäre 60 g Tri-n-butylzinnhydrid gegeben. Die Reaktionslösung wird 4 Stunden zum Rückfluss erwärmt und anschliessend am Vakuum eingeengt. Der kristalline Rückstand wird in Cyclohexan aufgenommen, abgenutscht und mit Aether gewaschen. Man erhält exo-6-(p-Chlorphenyl)-3-aza-bicyclo[3.1.$^{1,5}$.1$^{1,5}$]heptan-2,4-dion als weisse Kristalle vom Smp. 179-181°.

e) 3,5 g exo-6-(p-Chlorphenyl)-3-aza-bicyclo[3.1.$^{1,5}$.1$^{1,5}$]heptan-2,4-dion in 75 ml Toluol mit 24 ml

Natriumdihydrobis(2-methoxyäthoxy)-aluminat-Toluollösung (70 %; FLUKA) umgesetzt und mit 24 ml konzentrierter Natronlauge wie in Beispiel 4d2) angegeben aufgearbeitet. Man erhält exo-6-(p-Chlorphenyl)-3-aza-bicyclo[3.1.$^{1,5}$.1$^{1,5}$]heptan-hydrochlorid vom Smp. 201-203°.

Das als Ausgangsmaterial zu verwendende 1-Phenyl-3-aza-bicyclo-[3.1.$^{1,5}$.1$^{1,5}$]heptan(-hydrochlorid) kann folgendermassen hergestellt werden:

a2) Zu einer gerührten Lösung von 88,8 g 2-Phenylacrylsäure in 7 ml Dimethylformamid und 1,6 Liter Methylenchlorid wird 2 1/2 Stunden bei Raumtemperatur eine Lösung von 103 ml Oxalylchlorid in 400 ml Methylenchlorid getropft. Nach beendeter Zugabe wird 2 Stunden nachgerührt und anschliessend am Vakuum eingedampft. Das braune, ölige Produkt wird in 600 ml Aether aufgenommen und vom klebrigen Rückstand getrennt, über Diatomeenerde (HYFLO-Super-Cel®) filtriert und am Vakuum eingeengt. Das braune Oel wird in 0,8 Liter Methylenchlorid gelöst und in eine auf 0-5° gekühlte Lösung von 95,6 g N-(p-Methoxybenzyl)-acrylamid, 6,43 g 4-Di-methylaminopyridin und 63,1 g Triäthylamin in 1 Liter Methylenchlorid getropft. Nach beendeter Zugabe wird 3 Stunden bei Raumtemperatur nachgerührt. Nach dem Eindampfen der Reaktionslösung auf 250 ml wird 1 Liter Aether zugegeben. Die organische Phase wird vom klebrigen Rückstand abdekantiert. Der Rückstand wird 3 mal mit 500 ml Aether aufgenommen und die organische Phase jeweils abdekantiert. Die vereinigten organischen Phasen werden auf 200 ml eingeengt und über HYFLO-Super Cel® (s.o.) filtriert. Durch Eindampfen zur Trockne erhält man 4-Aza-4-(4-methoxybenzyl)-2-phenyl-1,6-heptadien-3,5-dion als braunes Oel. Dieses wird sofort weiterumgesetzt.

b2) Eine Lösung von 65,7 g 4-Aza-4-(p-methoxybenzyl)-2-phenyl-1,6-heptadien-3,5-dion und 0,5 g 2,6-Di-tert-butyl-p-kresol in 1 Liter 1,3-Dichlorbenzol wird 6 Stunden bei 170° gerührt. Nach dem Eindampfen wird der Rückstand mit Toluol-Aether (9:1) an 2,5 kg Kieselgel chromatographiert. Das erhaltene braune Oel wird bei 70° in 550 ml Diisopropyläther gelöst und unter Rühren im Eisbad abgekühlt. Der Niederschlag wird abgenutscht. Man erhält nach dem Trocknen am Hochvakuum 3-(4-Methoxybenzyl)-1-phenyl-3-aza-bicyclo[3.1.$^{1,5}$.1$^{1,5}$]heptan-2,4-dion als weisse Kristalle vom Smp. 87-88°.

c2) Zu einer gerührten Lösung von 53 g 3-(4-Methoxybenzyl)-1-phenyl-3-aza-bicyclo[3.1.$^{1,5}$.1$^{1,5}$]heptan-2,4-dion in 560 ml Acetonitril wird bei Raumtemperatur eine Lösung von 344 g Cer(IV)-ammoniumnitrat in 1,1 Liter Acetonitril zugetropft. Nach 1 Stunde werden 515 ml Wasser zugegeben und es wird 2 Stunden gerührt. Durch Abdestillieren von Acetonitril wird auf das halbe Volumen eingeengt und anschliessend mit 1 Liter Wasser verdünnt. Das ausgefallene Produkt wird abgenutscht, mit Wasser gewaschen und am Vakuum getrocknet. Die braungelbliche, kristalline Substanz wird in 800 ml Methylenchlorid aufgenommen, mit 11 ml n-Propylamin versetzt und über Nacht stehen gelassen. Die schwarze Lösung wird eingeengt und die braune, kristalline Masse mit 80 ml Methylenchlorid-Aether (1:1) versetzt, abgenutscht und am Hochvakuum getrocknet. Man erhält 1-Phenyl-3-aza-bicyclo-[3.1.$^{1,5}$.1$^{1,5}$]heptan-2,4-dion in Form weisser Kristalle vom Smp. 217-218°.

d2) Zur gerührten Suspension von 3 g 1-Phenyl-3-aza-bicyclo-[3.1.$^{1,5}$.1$^{1,5}$]heptan-2,4-dion in 150 ml Toluol werden unter Stickstoffatmosphäre 25,5 ml Natriumdihydrobis-(2-methoxyäthoxy)-aluminat-Toluollösung (70 %; FLUKA) zugetropft. Während der Zugabe wird die Temperatur durch externe Kühlung im Eisbad im Bereich von 25-35° gehalten. Nach beendeter Zugabe wird 15 Minuten bei Raumtemperatur nachgerührt und anschliessend 1 Stunde zum Rückfluss erwärmt. Nach dem Abkühlen im Eisbad werden bei 10-15° 25,5 ml konzentrierte Natronlauge zugetropft. Die organische Phase wird abdekantiert, und die Wasserphase mit Toluol gewaschen. Die vereinigten organischen Phasen werden zweimal mit 100 ml Wasser und einmal mit 70 ml gesättigter Kochsalzlösung gewaschen. Nach der Zugabe von Magnesiumsulfat wird die organische Phase filtriert und am Wasserstrahlvakuum eingeengt. Das bräunliche Oel wird in 50 ml Aether gelöst. Das 1-Phenyl-3-aza-bicyclo[3.1.$^{1,5}$.1$^{1,5}$]heptan-hydrochlorid wird durch Einleiten von Chlorwasserstoff als kristallines Produkt erhalten, welches nach dem Abnutschen nochmals in Aether aufgeschlämmt und wiederum abgenutscht und zuletzt am Hochvakuum über Nacht getrocknet wird: weisse Kristalle vom Smp. 248-249°.

Beispiel 5: Tabletten, enthaltend 75 mg Wirkstoff, z.B. 3-(4-Phenylpiperidino)-1-hydroxy-propan-1,1-diphosphonsäure oder ein Salz, z.B. das Natriumsalz, davon, können folgendermassen hergestellt werden:

Bestandteile (für 1000 Tabletten)

| | |
|---|---|
| Wirkstoff | 75,0 g |
| Lactose | 268,5 g |
| Maisstärke | 22,5 g |
| Polyäthylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann wird der Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend jeweils 75 mg einer anderen der in den Beispielen 1 bis 4 genannten Verbindungen der Formel I hergestellt werden, wobei diese auch in Form von Salzen mit Basen, z.B. als Dinatriumsalz, vorliegen können.

Beispiel 6: Tabletten, enthaltend 10 mg Wirkstoff, z.B. 3-(4-Phenylpiperidino)-1-hydroxy-propan-1,1-diphosphonsäure oder ein Salz, z.B. das Natriumsalz davon, können folgendermassen hergestellt werden:

Zusammensetzung (für 1000 Tabletten)

| | |
|---|---|
| Wirkstoff | 10,0 g |
| Lactose | 328,5 g |
| Maisstärke | 17,5 g |
| Polyäthylenglykol 6000 | 5,0 g |
| Talkum | 25,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyäthylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35° getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise können auch Tabletten, enthaltend 10 mg einer anderen Verbindung der Formel I gemäss den Beispielen 1 bis 4 hergestellt werden, wobei diese auch in Form von Salzen mit Basen, z.B. als Dinatriumsalz, vorliegen können.

Beispiel 7: Gelatinesteckkapseln, enthaltend 100 mg Wirkstoff, z.B. 3-(4-Phenylpiperidino)-1-hydroxy-propan-1,1-diphosphonsäure oder ein Salz, z.B. das Natriumsalz davon, können folgendermassen hergestellt werden:

Zusammensetzung (für 1000 Kapseln)

| | |
|---|---|
| Wirkstoff | 350,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem Wirkstoff (lyophilisiert) gesiebt und beide Komponenten 10 Minuten lang innig vermischt. Dann wird die mikrokristalline Cellulose durch ein Sieb mit einer Maschenweite von 0,9 mm hinzugesiebt und erneut 10 Minuten innig vermischt. Zuletzt wird das Magnesiumstearat durch ein Sieb mit einer Maschenweite von 0,8 mm hinzugesiebt und nach 3 Minuten weiteren Mischens je 390 mg der Mischung in Gelatinesteckkapseln der Grösse 0 (elongated) abgefüllt.

In analoger Weise können auch Kapseln, enthaltend 100 mg einer anderen Verbindung der Formel I gemäss den Beispielen 1 bis 4 hergestellt werden, wobei diese auch in Form von Salzen mit Basen, z.B. als Dinatriumsalz, vorliegen können.

Beispiel 8: Eine 0,2 %ige Injektions- bzw. Infusionslösung kann beispielsweise folgendermassen hergestellt werden.

Wirkstoff, z.B. 3-(4-Phenylpiperidino)-1-hydroxy-propan-1,1-diphosphonsäure oder ein Salz,

| | |
|---|---|
| z.B. das Natriumsalz davon | 5,0 g |
| Natriumchlorid | 22,5 g |
| Phosphatpuffer pH = 7,4 | 300,0 g |
| Wasser, entmin. | ad 2500,0 ml |

Der Wirkstoff wird in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen (enthaltend je 2,0 bzw. 5,0 mg Wirkstoff abgefüllt.

## Ansprüche

1. Aromatisch substituierte Azacycloalkylalkandiphosphonsäuren der Formel

$$R - alk - \underset{PO_3H_2}{\overset{PO_3H_2}{\underset{|}{\overset{|}{C}}}} - OH \qquad (I)$$

worin R einen durch einen unsubstituierten oder durch $C_1$-$C_4$-Alkyl, $C_1$-$C_4$-Alkoxy und/oder Halogen der Atomnummer bis und mit 35 mono- oder disubstituierten Phenyl-, Pyridyl- oder Thienylrest R' substituierten Pyrrolidino-, Piperidino-, Piperazino-, 3-Aza-bicyclo[$3.1^{1,5}.1^{1,5}$]hept-3-yl-oder Octahydroazocinorest R" bedeutet und alk für geradkettiges $C_2$-$C_5$-Alkylen steht, und ihre Salze.

2. Verbindungen gemäss Anspruch 1 der Formel I, worin R einen 4-R'-Piperidinorest oder 6-R'-3-Aza-bicyclo[$3.1^{1,5}.1^{1,5}$]hept-3-ylrest bedeutet, R' unsubstituiertes oder durch $C_1$-$C_4$-Alkyl, $C_1$-$C_4$-Alkoxy oder Halogen der Atomnummer bis und mit 35 substituiertes Phenyl darstellt und alk geradkettiges $C_2$-$C_5$-Alkylen der Formel $-(CH_2)_n-$, worin n für eine ganze Zahl von 2 bis und mit 5 steht, bedeutet, und ihre Salze.

3. Verbindungen gemäss Anspruch 1 der Formel I, worin R einen 3-R'-Pyrrolidino- oder 3- oder 4-R'-Piperidinorest bedeutet, R' unsubstituiertes oder durch $C_1$-$C_4$-Alkyl, $C_1$-$C_4$-Alkoxy oder Halogen der Atomnummer bis und mit 35 substituiertes Phenyl darstellt und alk geradkettiges $C_2$-$C_5$-Alkylen der Formel $-(CH_2)_n-$, worin n für eine ganze Zahl von 2 bis und mit 5 steht, bedeutet, und ihre Salze.

**4.** 3-(4-Phenylpiperidino)-1-hydroxy-propan-1,1-diphosphonsäure gemäss Anspruch 1 oder ein Salz davon.

**5.** 3-(3-Phenylpyrrolidino)-1-hydroxy-propan-1,1-diphosphonsäure oder ein Salz davon.

**6.** 3-[4-(p-Methoxyphenyl)piperidino]-1-hydroxy-propan-1,1-diphosphonsäure oder ein Salz davon.

**7.** 3-[4-(p-Chlorphenyl)piperidino]-1-hydroxy-propan-1,1-diphosphonsäure oder ein Salz davon.

**8.** 3-[4-(p-Fluorphenyl)piperidino]-1-hydroxy-propan-1,1-diphosphonsäure oder ein Salz davon.

**9.** 4-(4-Phenylpiperidino)-1-hydroxy-butan-1,1-diphosphonsäure oder ein Salz davon.

**10.** 3-[6-endo-(p-Chlorphenyl)-3-aza-bicyclo[$3.1^{1,5}.1^{1,5}$]hept-3-yl]-1-hydroxy-propan-1,1-diphosphonsäure gemäss Anspruch 1 oder ein Salz davon.

**11.** 6-(4-Phenylpiperidino)-1-hydroxy-hexan-1,1-diphosphonsäure oder ein Salz davon.

**12.** 3-[4-(p-Methylphenyl)piperidino]-1-hydroxy-propan-1,1-diphosphonsäure oder ein Salz davon.

**13.** 3-(4-Phenyl-octahydro-azocino)-1-hydroxy-propan-1,1-diphosphonsäure oder ein Salz davon.

**14.** 3-[4-(m-Fluorphenyl)piperidino]-1-hydroxy-propan-1,1-diphosphonsäure oder ein Salz davon.

**15.** 3-(1-Phenylpiperazino)-1-hydroxy-propan-1,1-diphosphonsäure oder ein Salz davon.

**16.** 3-[3-(p-Chlorphenyl)pyrrolidino]-1-hydroxy-propan-1,1-diphosphonsäure oder ein Salz davon.

**17.** 3-[6-exo(p-Chlorphenyl)-3-aza-bicyclo[$3.1^{1,5}.1^{1,5}$]hept-3-yl]-1-hydroxy-propan-1,1-diphosphonsäure oder ein Salz davon.

**18.** 3-(1-Phenyl-3-aza-bicyclo[$3.1^{1,5}.1^{1,5}$]hept-3-yl)-1-hydroxy-propan-1,1-diphosphonsäure oder ein Salz davon.

**19.** Eine Verbindung gemäss einem der Ansprüche 2 und 11-18 zur therapeutischen Behandlung des menschlichen oder tierischen Körpers, z.B. als den Calciumstoffwechsel regulierendes Mittel.

**20.** Eine Verbindung gemäss einem der Ansprüche 1 und 3-10 zur therapeutischen Behandlung des menschlichen oder tierischen Körpers, z.B. als den Calciumstoffwechsel regulierendes Mittel.

**21.** Pharmazeutisches Präparat, enthaltend eine Verbindung gemäss einem der Ansprüche 2 und 11-18 neben üblichen pharmazeutischen Hilfsstoffen.

**22.** Pharmazeutisches Präparat, enthaltend eine Verbindung gemäss einem der Ansprüche 1, 3-10 und 19 neben üblichen pharmazeutischen Hilfsstoffen.

**23.** Verfahren zur Herstellung aromatisch substituierte Azacycloalkylalkandiphosphonsäuren gemäss Anspruch 1 und ihrer Salze, dadurch gekennzeichnet, dass man

a) in einer Verbindung der Formel

$$R - alk - \overset{X_1}{\underset{X_2}{C}} - OH \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder
b) eine Verbindung der Formel

$$H_2N - alk - \begin{array}{c} PO_3H_2 \\ | \\ C \\ | \\ PO_3H_2 \end{array} - OH \qquad (III)$$

oder ein Salz davon mit einem reaktiven Ester eines entsprechenden aromatisch substituierten aliphatischen Dialkohols umsetzt oder
c) eine Verbindung der Formel

R - alk - $X_3$ (IV),

worin $X_3$ Carboxy, Carbamoyl oder Cyano bedeutet, mit phosphoriger Säure und Phosphortrichlorid umsetzt, das Primärprodukt hydrolysiert und in einem ausgehend von Verbindungen der Formel IV, worin $X_3$ Cyano oder Carbamoyl ist, erhaltenen Zwischenprodukt der Formel

$$R - alk - \begin{array}{c} PO_3H_2 \\ | \\ C \\ | \\ PO_3H_2 \end{array} - NH_2 \qquad (V)$$

bzw. einem Salz davon die Aminogruppe durch Behandlung mit salpetriger Säure durch Hydroxy ersetzt und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

**24.** Verwendung von Verbindungen gemäss einem der Ansprüche 1-18 zur Herstellung eines den Calciumstoffwechsel regulierenden Mittels.

Patentansprüche für folgende Vertragsstaaten : ES, GR

**1.** Verfahren zur Herstellung aromatisch substituierte Azacycloalkylalkandiphosphonsäuren der Formel

$$R - alk - \begin{array}{c} PO_3H_2 \\ | \\ C \\ | \\ PO_3H_2 \end{array} - OH \qquad (I)$$

worin R einen durch einen unsubstituierten oder durch $C_1$-$C_4$-Alkyl, $C_1$-$C_4$-Alkoxy und/oder Halogen der Atomnummer bis und mit 35 mono- oder disubstituierten Phenyl-, Pyridyl- oder Thienylrest R' substituierten Pyrrolidino-, Piperidino-, Piperazino-, 3-Aza-bicyclo[$3.1^{1,5}.1^{1,5}$]hept-3-yl-oder Octahydroazocinorest R" bedeutet und alk für geradkettiges $C_2$-$C_5$-Alkylen steht, und ihrer Salze, dadurch gekennzeichnet, dass man
a) in einer Verbindung der Formel

$$R - alk - \begin{array}{c} X_1 \\ | \\ C \\ | \\ X_2 \end{array} - OH \qquad (II),$$

worin $X_1$ eine funktionell abgewandelte und $X_2$ eine freie oder funktionell abgewandelte Phosphonogruppe bedeutet, $X_1$ und gegebenenfalls $X_2$ in die freie Phosphonogruppe überführt oder
b) eine Verbindung der Formel

$$H_2N - alk - \underset{PO_3H_2}{\overset{PO_3H_2}{C}} - OH \qquad (III)$$

oder ein Salz davon mit einem reaktiven Ester eines entsprechenden aromatisch substituierten aliphatischen Dialkohols umsetzt oder
c) eine Verbindung der Formel

R - alk - $X_3$ (IV),

worin $X_3$ Carboxy, Carbamoyl oder Cyano bedeutet, mit phosphoriger Säure und Phosphortrichlorid umsetzt, das Primärprodukt hydrolysiert und in einem ausgehend von Verbindungen der Formel IV, worin $X_3$ Cyano oder Carbamoyl ist, erhaltenen Zwischenprodukt der Formel

$$R - alk - \underset{PO_3H_2}{\overset{PO_3H_2}{C}} - NH_2 \qquad (V)$$

bzw. einem Salz davon die Aminogruppe durch Behandlung mit salpetriger Säure durch Hydroxy ersetzt und gewünschtenfalls eine erhaltene Verbindung in eine andere Verbindung der Formel I und/oder eine erhaltene freie Verbindung in ein Salz oder ein erhaltenes Salz in die freie Verbindung oder in ein anderes Salz überführt.

2. Verfahren gemäss Anspruch 1 zur Herstellung von Verbindungen der Formel I, worin R einen 4-R'-Piperidinorest oder 6-R'-3-Aza-bicyclo-[$3.1^{1,5}.1^{1,5}$]hept-3-ylrest bedeutet, wobei R' unsubstituiertes oder durch $C_1$-$C_4$-Alkyl, $C_1$-$C_4$-Alkoxy oder Halogen der Atomnummer bis und mit 35 substituiertes Phenyl darstellt, und alk geradkettiges $C_2$-$C_5$-Alkylen der Formel $-(CH_2)_n-$, worin n für eine ganze Zahl von 2 bis und mit 5 steht, bedeutet, und ihrer Salze.

3. Verfahren gemäss Anspruch 1 zur Herstellung von Verbindungen der Formel I, worin R einen 3-R'-Pyrrolidino- oder 3- oder 4-R'-Piperidinorest bedeutet, wobei R' unsubstituiertes oder durch $C_1$-$C_4$-Alkyl, $C_1$-$C_4$-Alkoxy oder Halogen der Atomnummer bis und mit 35 substituiertes Phenyl darstellt, und alk geradkettiges $C_2$-$C_5$-Alkylen der Formel $-(CH_2)_n-$, worin n für eine ganze Zahl von 2 bis und mit 5 steht, bedeutet, und ihrer Salze.

4. Verfahren gemäss Anspruch 1 zur Herstellung von 3-(4-Phenylpiperidino)-1-hydroxy-propan-1,1-diphosphonsäure oder eines Salzes davon.

5. Verfahren gemäss Anspruch 1 zur Herstellung von 3-(3-Phenylpyrrolidino)-1-hydroxy-propan-1,1-diphosphonsäure oder eines Salzes davon.

6. Verfahren gemäss Anspruch 1 zur Herstellung von 3-[4-(p-Methoxyphenyl)piperidino]-1-hydroxy-propan-1,1-diphosphonsäure oder eines Salzes davon.

7. Verfahren gemäss Anspruch 1 zur Herstellung von 3-[4-(p-Chlorphenyl)piperidino]-1-hydroxy-propan-1,1-diphosphonsäure oder eines Salzes davon.

8. Verfahren gemäss Anspruch 1 zur Herstellung von 3-[4-(p-Fluorphenyl)piperidino]-1-hydroxy-propan-1,1-diphosphonsäure oder eines Salzes davon.

9. Verfahren gemäss Anspruch 1 zur Herstellung von 4-(4-Phenylpiperidino)-1-hydroxy-butan-1,1-diphosphonsäure oder eines Salzes davon.

10. Verfahren gemäss Anspruch 1 zur Herstellung von 3-[6-endo-(p-Chlorphenyl)-3-aza-bicyclo[$3.1^{1,5}.1^{1,5}$]-hept-3-yl]-1-hydroxy-propan-1,1-diphosphonsäure oder eines Salzes davon.

11. Verfahren gemäss Anspruch 1 zur Herstellung von 6-(4-Phenylpiperidino)-1-hydroxy-hexan-1,1-diphosphonsäure oder eines Salzes davon.

12. Verfahren gemäss Anspruch 1 zur Herstellung von 3-[4-(p-Methylphenyl)piperidino]-1-hydroxy-propan-1,1-diphosphonsäure oder eines Salzes davon.

13. Verfahren gemäss Anspruch 1 zur Herstellung von 3-(4-Phenyl-octahydro-azocino)-1-hydroxy-propan-1,1-diphosphonsäure oder eines Salzes davon.

14. Verfahren gemäss Anspruch 1 zur Herstellung von 3-[4-(m-Fluorphenyl)piperidino]-1-hydroxy-propan-1,1-diphosphonsäure oder eines Salzes davon.

15. Verfahren gemäss Anspruch 1 zur Herstellung von 3-(1-Phenylpiperazino)-1-hydroxy-propan-1,1-diphosphonsäure oder eines Salzes davon.

16. Verfahren gemäss Anspruch 1 zur Herstellung von 3-[3-(p-Chlorphenyl)pyrrolidino]-1-hydroxy-propan-1,1-diphosphonsäure oder eines Salzes davon.

17. Verfahren gemäss Anspruch 1 zur Herstellung von 3-[6-exo(p-Chlorphenyl)-3-aza-bicyclo[3.$1^{1,5}$.$1^{1,5}$]-hept-3-yl]-1-hydroxy-propan-1,1-diphosphonsäure oder eines Salzes davon.

18. Verfahren gemäss Anspruch 1 zur Herstellung von 3-(1-Phenyl-3-aza-bicyclo[3.$1^{1,5}$.$1^{,15}$]hept-3-yl)-1-hydroxy-propan-1,1-diphosphonsäure oder eines Salzes davon.

19. Verfahren zur Herstellung pharmazeutischer Präparate, dadurch gekennzeichnet, dass man eine Verbindung, erhältlich gemäss einem der Ansprüche 1-18 mit üblichen pharmazeutischen Hilfsstoffen vermischt.

## Claims

1. Aromatically substituted azacycloalkylalkanediphosphonic acids of the formula

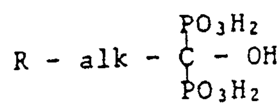

(I)

in which R represents a pyrrolidino, piperidino, piperazino, 3-aza-bicyclo[3.$1^{1,5}$.$1^{1,5}$]hept-3-yl or octahydroazocino radical R" substituted by a phenyl, pyridyl or thienyl radical R' that is unsubstituted or is mono- or di-substituted by $C_1$-$C_4$alkyl, $C_1$-$C_4$alkoxy and/or by halogen having an atomic number of up to and including 35, and alk represents straight-chain $C_2$-$C_5$alkylene, and their salts.

2. Compounds according to claim 1 of the formula I in which R represents a 4-R'-piperidino or 6-R'-3-aza-bicyclo[3.$^{1,5}$.$1^{1,5}$]hept-3-yl radical, R' represents unsubstituted phenyl or phenyl substituted by $C_1$-$C_4$alkyl, $C_1$-$C_4$alkoxy or by halogen having an atomic number of up to and including 35, and alk represents straight-chain $C_2$-$C_5$alkylene of the formula $-(CH2)_n-$ wherein n represents an integer from 2 up to and including 5, and their salts.

3. Compounds according to claim 1 of the formula I in which R represents a 3-R'-pyrrolidino or 3- or 4-R'-piperidino radical, R' represents unsubstituted phenyl or phenyl substituted by $C_1$-$C_4$alkyl, $C_1$-$C_4$alkoxy or by halogen having an atomic number of up to and including 35, and alk represents straight-chain $C_2$-$C_5$alkylene of the formula $-(CH2)_n-$ wherein n represents an integer from 2 up to and including 5, and their salts.

4. 3-(4-phenylpiperidino)-1-hydroxy-propane-1,1-diphosphonic acid according to claim 1 or a salt thereof.

5. 3-(3-phenylpyrrolidino)-1-hydroxy-propane-1,1-diphosphonic acid or a salt thereof.

**6.** 3-[4-(p-methoxyphenyl)piperidino]-1-hydroxy-propane-1,1-diphosphonic acid or a salt thereof.

**7.** 3-[4-(p-chlorophenyl)piperidino]-1-hydroxy-propane-1,1-diphosphonic acid or a salt thereof.

**8.** 3-[4-(p-fluorophenyl)piperidino]-1-hydroxy-propane-1,1-diphosphonic acid or a salt thereof.

**9.** 4-(4-phenylpiperidino)-1-hydroxy-butane-1,1-diphosphonic acid or a salt thereof.

**10.** 3-[6-endo-(p-chlorophenyl)-3-aza-bicyclo-$[3.1^{1,5}.1^{1,5}]$hept-3-yl]-1-hydroxy-propane-1,1-diphosphonic acid according to claim 1 or a salt thereof.

**11.** 6-(4-phenylpiperidino)-1-hydroxy-hexane-1,1-diphosphonic acid or a salt thereof.

**12.** 3-[4-(p-methylphenyl)piperidino]-1-hydroxy-propane-1,1-diphosphonic acid or a salt thereof.

**13.** 3-(4-phenyl-octahydro-azocino)-1-hydroxy-propane-1,1-diphosphonic acid or a salt thereof.

**14.** 3-[4-(m-fluorophenyl)piperidino]-1-hydroxy-propane-1,1-diphosphonic acid or a salt thereof.

**15.** 3-(1-phenylpiperazino)-1-hydroxy-propane-1,1-diphosphonic acid or a salt thereof.

**16.** 3-[3-(p-chlorophenyl)pyrrolidino]-1-hydroxy-propane-1,1-diphosphonic acid or a salt thereof.

**17.** 3-[6-exo-(p-chlorophenyl)-3-aza-bicyclo-$[3.1^{1,5}.1^{1,5}]$hept-3-yl]-1-hydroxy-propane-1,1-diphosphonic acid or a salt thereof.

**18.** 3-(1-phenyl-3-aza-bicyclo$[3.1^{1,5}.1^{1,5}]$hept-3-yl)-1-hydroxy-propane-1,1-diphosphonic acid or a salt thereof.

**19.** A compound according to any one of claims 2 and 11 to 18 for the therapeutic treatment of the human or animal body, for example as an agent that regulates the calcium metabolism.

**20.** A compound according to any one of claims 1 and 3 to 10 for the therapeutic treatment of the human or animal body, for example as an agent that regulates the calcium metabolism.

**21.** A pharmaceutical preparation containing a compound according to any one of claims 2 and 11 to 18 together with customary pharmaceutical adjuncts.

**22.** A pharmaceutical preparation containing a compound according to any one of claims 1, 3 to 10 and 19 together with customary pharmaceutical adjuncts.

**23.** A process for the manufacture of aromatically substituted azacycloalkylalkanediphosphonic acids according to claim 1 and their salts, characterised in that

a) in a compound of the formula

$$R - alk - \underset{X_2}{\overset{X_1}{C}} - OH \qquad (II),$$

in which $X_1$ represents a functionally modified phosphono group and $X_2$ represents a free or functionally modified phosphono group, $X_1$ and, where appropriate, $X_2$ is(are) converted into the free phosphono group, or

b) a compound of the formula

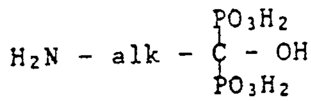 (III)

or a salt thereof is reacted with a reactive ester of a corresponding aromatically substituted aliphatic dialcohol, or

c) a compound of the formula

R - alk - $X_3$ (IV),

in which $X_3$ represents carboxy, carbamoyl or cyano, is reacted with phosphorous acid and phosphorus trichloride, the primary product is hydrolysed and, in an intermediate of the formula

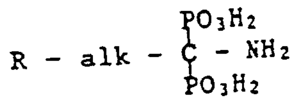 (V)

obtained starting from compounds of the formula IV in which $X_3$ represents cyano or carbamoyl, or in a salt thereof, the amino group is replaced by hydroxy by treatment with nitrous acid, and, if desired, a resulting compound is converted into a different compound of the formula I and/or a resulting free compound is converted into a salt or a resulting salt is converted into the free compound or into a different salt.

**24.** The use of compounds according to any one of claims 1 to 18 for the manufacture of a composition that regulates the calcium metabolism.

Claims for the following Contracting States : ES and GR

**1.** A process for the manufacture of aromatically substituted azacycloalkylalkanediphosphonic acids of the formula

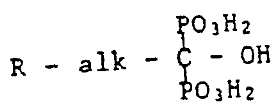 (I)

in which R represents a pyrrolidino, piperidino, piperazino, 3-aza-bicyclo[$3.1^{1,5}.1^{1,5}$]hept-3-yl or octahydroazocino radical R" substituted by a phenyl, pyridyl or thienyl radical R' that is unsubstituted or is mono- or di-substituted by $C_1$-$C_4$alkyl, $C_1$-$C_4$alkoxy and/or by halogen having an atomic number of up to and including 35, and alk represents straight-chain $C_2$-$C_5$alkylene, and their salts, characterised in that

a) in a compound of the formula

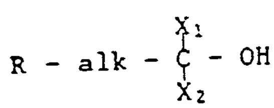 (II),

in which $X_1$ represents a functionally modified phosphono group and $X_2$ represents a free or functionally modified phosphono group, $x_1$ and, where appropriate, $X_2$ is(are) converted into the free phosphono group, or

b) a compound of the formula

$$H_2N - alk - \underset{PO_3H_2}{\overset{PO_3H_2}{\underset{|}{\overset{|}{C}}}} - OH \qquad (III)$$

or a salt thereof is reacted with a reactive ester of a corresponding aromatically substituted aliphatic dialcohol, or
c) a compound of the formula

R - alk - $X_3$ (IV),

in which $X_3$ represents carboxy, carbamoyl or cyano, is reacted with phosphorous acid and phosphorus trichloride, the primary product is hydrolysed and, in an intermediate of the formula

$$R - alk - \underset{PO_3H_2}{\overset{PO_3H_2}{\underset{|}{\overset{|}{C}}}} - NH_2 \qquad (V)$$

obtained starting from compounds of the formula IV in which $X_3$ represents cyano or carbamoyl, or in a salt thereof, the amino group is replaced by hydroxy by treatment with nitrous acid, and, if desired, a resulting compound is converted into a different compound of the formula I and/or a resulting free compound is converted into a salt or a resulting salt is converted into the free compound or into a different salt.

**2.** A process according to claim 1 for the manufacture of compounds of the formula I in which R represents a 4-R'-piperidino or 6-R'-3-aza-bicyclo[$3.1^{1,5}.1^{1,5}$]hept-3-yl radical, wherein R' represents unsubstituted phenyl or phenyl substituted by $C_1$-$C_4$alkyl, $C_1$-$C_4$alkoxy or by halogen having an atomic number of up to and including 35, and alk represents straight-chain $C_2$-$C_5$alkylene of the formula -(CH2)n- wherein n represents an integer from 2 up to and including 5, and their salts.

**3.** A process according to claim 1 for the manufacture of compounds of the formula I in which R represents a 3-R'-pyrrolidino or 3- or 4-R'-piperidino radical, wherein R' represents unsubstituted phenyl or phenyl substituted by $C_1$-$C_4$alkyl, $C_1$-$C_4$alkoxy or by halogen having an atomic number of up to and including 35, and alk represents straight-chain $C_2$-$C_5$alkylene of the formula -(CH2)n-wherein n represents an integer from 2 up to and including 5, and their salts.

**4.** A process according to claim 1 for the manufacture of 3-(4-phenylpiperidino)-1-hydroxy-propane-1,1-diphosphonic acid or a salt thereof.

**5.** A process according to claim 1 for the manufacture of 3-(3-phenylpyrrolidino)-1-hydroxy-propane-1,1-diphosphonic acid or a salt thereof.

**6.** A process according to claim 1 for the manufacture of 3-[4-(p-methoxyphenyl)piperidino]-1-hydroxy-propane-1,1-diphosphonic acid or a salt thereof.

**7.** A process according to claim 1 for the manufacture of 3-[4-(p-chlorophenyl)piperidino]-1-hydroxy-propane-1,1-diphosphonic acid or a salt thereof.

**8.** A process according to claim 1 for the manufacture of 3-[4-(p-fluorophenyl)piperidino]-1-hydroxy-propane-1,1-diphosphonic acid or a salt thereof.

**9.** A process according to claim 1 for the manufacture of 4-(4-phenylpiperidino)-1-hydroxy-butane-1,1-diphosphonic acid or a salt thereof.

**10.** A process according to claim 1 for the manufacture of 3-[6-endo-(p-chlorophenyl)-3-aza-bicyclo-[$3.1^{1,5}.1^{1,5}$]hept-3-yl]-1-hydroxy-propane-1,1-diphosphonic acid or a salt thereof.

**11.** A process according to claim 1 for the manufacture of 6-(4-phenylpiperidino)-1-hydroxy-hexane-1,1-diphosphonic acid or a salt thereof.

**12.** A process according to claim 1 for the manufacture of 3-[4-(p-methylphenyl)piperidino]-1-hydroxy-propane-1,1-diphosphonic acid or a salt thereof.

**13.** A process according to claim 1 for the manufacture of 3-(4-phenyl-octahydro-azocino)-1-hydroxy-propane-1,1-diphosphonic acid or a salt thereof.

**14.** A process according to claim 1 for the manufacture of 3-[4-(m-fluorophenyl)piperidino]-1-hydroxy-propane-1,1-diphosphonic acid or a salt thereof.

**15.** A process according to claim 1 for the manufacture of 3-(1-phenylpiperazino)-1-hydroxy-propane-1,1-diphosphonic acid or a salt thereof.

**16.** A process according to claim 1 for the manufacture of 3-[3-(p-chlorophenyl)pyrrolidino]-1-hydroxy-propane-1,1-diphosphonic acid or a salt thereof.

**17.** A process according to claim 1 for the manufacture of 3-[6-exo-(p-chlorophenyl)-3-aza-bicyclo-$[3.1^{1,5}.1^{1,5}]$hept-3-yl]-1-hydroxy-propane-1,1-diphosphonic acid or a salt thereof.

**18.** A process according to claim 1 for the manufacture of 3-(1-phenyl-3-aza-bicyclo$[3.1^{1,5}.1^{1,5}]$hept-3-yl)-1-hydroxy-propane-1,1-diphosphonic acid or a salt thereof.

**19.** A process for the manufacture of pharmaceutical preparations, characterised in that a compound obtainable according to any one of claims 1 to 18 is mixed with customary pharmaceutical adjuncts.

**Revendications**

**1.** Acides azacycloalkylalcanediphosphoniques substitués aromatiquement de formule

$$R - alk - \underset{\displaystyle PO_3H_2}{\overset{\displaystyle PO_3H_2}{\underset{|}{\overset{|}{C}}}} - OH \qquad (I)$$

dans laquelle R représente un reste R" pyrrolidino-, pipéridino-, pipérazino-, 3-aza-bicyclo$[3.1^{1,5}.1^{1,5}]$ heptyl-3 ou octahydroazocino substitué par un reste R' phényle, pyridyle ou thiényle non substitué ou mono-ou disubstitué par un alkyle $C_1$-$C_4$, alcoxy $C_1$-$C_4$ et/ou halogène de numéro atomique jusqu'à 35 compris et alk est un alkylène $C_2$-$C_5$ linéaire, et leurs sels.

**2.** Composés selon la revendication 1 de formule I, dans laquelle R est un reste 4-R'-pipéridino ou 6-R'3-aza-bicyclo$[3.1^{1,5}.1^{1,5}]$heptyl-3, R' un phényle non substitué ou substitué par un alkyle $C_1$-$C_4$, alcoxy $C_1$-$C_4$ ou halogène de numéro atomique jusqu'à 35 compris et alk est un alkylène $C_2$-$C_5$ linéaire de formule $-(CH_2)n-$, dans lequel n représente un nombre entier de 2 à 5 compris et leurs sels.

**3.** Composés selon la revendication 1 de formule I, dans laquelle R est un reste 3-R'-pyrrolidino ou 3- ou 4-R'-pipéridino, R' est un phényle non substitué ou substitué par un alkyle $C_1$-$C_4$, alcoxy $C_1$-$C_4$ ou halogène de numéro atomique jusqu'à 35 compris et alk est un alkylène $C_2$-$C_5$ linéaire de formule $-(CH_2)n-$, dans laquelle n est un nombre entier de 2 à 5 compris, et leurs sels.

**4.** Acide 3-(4-phénylpipéridino)-1-hydroxy-propane-1,1-diphosphonique selon la revendication 1 ou un de ses sels.

**5.** Acide 3-(3-phénylpyrrolidino)-1-hydroxy-propane-1,1-diphosphonique ou un de ses sels.

**6.** Acide 3-[4-(p-méthtoxyphényl)pipéridino]-1-hydroxy-propane-1,1-diphosphonique ou un de ses sels.

**7.** Acide 3-[4-(p-chlorophényl)pipéridino]-1-hydroxy-propane-1,1-diphosphonique ou un de ses sels.

**8.** Acide 3-[4-(p-fluoropnényl)pipéridino]-1-hydroxy-propane-1,1-diphosphonique ou un de ses sels.

**9.** Acide 4-(4-phénylpipéridino)-1-hydroxy-butane-1,1-diphosphonique ou un de ses sels.

**10.** Acide 3-[6-endo-(p-chlorophényl)-3-aza-bicyclo[3.$1^{1,5}$.$1^{1,5}$]heptyl-3]-1-hydroxy-propane-1,1-diphosphonique selon la revendication 1 ou un de ses sels.

**11.** Acide 6-(4-phénylpipéridino)-1-hydroxy-hexane-1,1-diphosphonique ou un de ses sels.

**12.** Acide 3-[4-(p-méthylphényl)pipéridino]-1-hydroxy-propane-1,1-diphosphonique ou un de ses sels.

**13.** Acide 3-(4-phényl-octahydro-azocino)-1-hydroxy-propane-1,1-diphosphonique ou un de ses sels.

**14.** Acide 3-[4-(m-fluorophényl)pipéridino]-1-hydroxy-propane-1,1-diphosphonique ou un de ses sels.

**15.** Acide 3-(1-phénylpipérazino)-1-hydroxy-propane-1,1-diphosphonique ou un de ses sels.

**16.** Acide 3-[3-(p-chlorophényl)pyrrolidino]-1-hydroxy-propane-1,1-diphosphonique ou un de ses sels.

**17.** Acide 3-[6-exo-(p-chlorophényl)-3-aza-bicyclo[3.$1^{1,5}$.$1^{1,5}$]heptyl-3]-1-hydroxy-propane-1,1-diphosphonique ou un de ses sels.

**18.** Acide 3-(1-phényl-3-aza-bicyclo[3.$1^{1,5}$.$1^{1,5}$]heptyl-3)-1-hydroxy-propane-1,1-diphosphonique ou un de ses sels.

**19.** Composé selon l'une des revendications 2 et 11 à 18 pour le traitement thérapeutique du corps humain ou animal, par exemple en tant qu'agent régulateur de l'échange de calcium.

**20.** Composé selon l'une des revendications 1 et 3 à 10 pour le traitement thérapeutique du corps humain ou animal, par exemple en tant qu'agent régulateur de l'échange de calcium.

**21.** Préparation pharmaceutique comprenant un composé selon l'une des revendications 2 et 11-18 en plus des additifs pharmaceutiques usuels.

**22.** Préparation pharmaceutique comprenant un composé selon l'une des revendications 1, 3 à 10 et 19 en plus des additifs pharmaceutiques usuels.

**23.** Procédé de préparation d'acides azacycloalkylalcanediphosphoniques selon la revendication 1 et leurs sels, caractérisé en ce que

a) dans un composé de formule

$$R - alk - \overset{\displaystyle X_1}{\underset{\displaystyle X_2}{C}} - OH \qquad (II),$$

dans laquelle $X_1$ est un groupe phosphono fonctionnellement dérivé et $X_2$ un groupe phosphono libre ou fonctionnellement dérivé, on transforme $X_1$ et le cas échéant $X_2$ en le groupe libre phosphono, ou

b) on fait réagir un composé de formule

$$H_2N - alk - \overset{\displaystyle PO_3H_2}{\underset{\displaystyle PO_3H_2}{C}} - OH \qquad (III)$$

ou un de ses sels avec un ester réactif d'un dialcool aliphatique substitué aromatiquement correspondant ou
c) on fait réagir un composé de formule

R - alk - $X_3$ (IV),

dans laquelle $X_3$ est un carboxy, carbamyle ou cyano, avec de l'acide phosphoreux et du trichlorure de phosphore, on hydrolyse le produit primaire, et dans le produit intermédiaire de formule

$$R - alk - \begin{array}{c} PO_3H_2 \\ | \\ C \\ | \\ PO_3H_2 \end{array} - NH_2 \qquad (V)$$

obtenu à partir de composés de formule IV, dans lesquels $X_3$ est cyano ou carbamyle, ou un de ses sels, on remplace le groupe amino par un hydroxy par traitement avec l'acide nitreux et si on le souhaite, on transforme le composé obtenu en un autre composé de formule I et/ou on transforme le composé libre obtenu en un sel ou on transforme un sel obtenu en le composé libre ou un autre sel.

**24.** Utilisation de composés selon l'une des revendications 1-18 pour produire un agent de régulation de l'échange de calcium.

Revendications pour les Etats Contractants Suivants : ES et GR

**1.** Procédé de préparation d'acides azacycloalkylalcanediphosphoniques aromatiquement substitués de formule

$$R - alk - \begin{array}{c} PO_3H_2 \\ | \\ C \\ | \\ PO_3H_2 \end{array} - OH \qquad (I)$$

dans laquelle R représente un reste R" pyrrolidino-, pipéridino-, pipérazino-, 3-aza-bicyclo[$3.1^{1,5}.1^{1,5}$]-heptyl-3 ou octahydroazocino substitué par un reste R' phényle, pyridyle ou thiényle non substitué ou mono- ou disubstitué par un alkyle $C_1$-$C_4$, alcoxy $C_1$-$C_4$ et/ou halogène de numéro atomique jusqu'à 35 compris et alk est un alkylène $C_2$-$C_5$ linéaire, et leurs sels, caractérisé en ce que
a) dans un composé de formule

$$R - alk - \begin{array}{c} X_1 \\ | \\ C \\ | \\ X_2 \end{array} - OH \qquad (II),$$

dans laquelle $X_1$ est un groupe phosphono fonctionnellement dérivé et $X_2$ un groupe phosphono libre ou fonctionnellement dérivé, on transforme $X_1$ et le cas échéant $X_2$ en le groupe libre phosphono, ou
b) on fait réagir un composé de formule

$$H_2N - alk - \begin{array}{c} PO_3H_2 \\ | \\ C \\ | \\ PO_3H_2 \end{array} - OH \qquad (III)$$

ou un de ses sels avec un ester réactif d'un dialcool aliphatique substitué aromatiquement correspondant ou
c) on fait réagir un composé de formule

R - alk - $X_3$ (IV),

dans laquelle $x_3$ est un carboxy, carbamyle ou cyano, notamment carboxy ou cyano, avec de l'acide phosphoreux et du trichlorure de phosphore, on hydrolyse le produit primaire, et dans le produit intermédiaire de formule

$$\begin{array}{ccc} & PO_3H_2 & \\ & | & \\ R - alk - & C & - NH_2 \\ & | & \\ & PO_3H_2 & \end{array} \qquad (V)$$

obtenu à partir de composés de formule IV, dans lesquels $X_3$ est cyano ou carbamyle, ou un de ses sels, on remplace le groupe amino par un hydroxy par traitement avec l'acide nitreux et, si on le souhaite, on transforme le composé obtenu en un autre composé de formule I et/ou on transforme le composé libre obtenu en un sel, ou on transforme un sel obtenu en le composé libre ou en un autre sel.

**2.** Procédé selon la revendication 1 de préparation de composés de formule I, dans laquelle R est un reste 4-R'-pipéridino ou 6-R'-3-aza-bicyclo[$3.1^{1,5}.1^{1,5}$]-heptyl-3, R' un phényle non substitué ou substitué par un alxyle $C_1$-$C_4$, alcoxy $C_1$-$C_4$ ou halogène de numéro atomique jusqu'à 35 compris et alk est un alkylène $C_2$-$C_5$ linéaire de formule $-(CH_2)n-$, dans lequel n représente un nombre entier de 2 à 5 compris et leurs sels.

**3.** Procédé selon la revendication 1 de préparation de composés de formule I, dans laquelle R est un reste 3-R'-pyrrolidino ou 3- ou 4-R'-pipéridino, R' est un phényle non substitué ou substitué par un alkyle $C_1$-$C_4$, alcoxy $C_1$-$C_4$ ou halogène de numéro atomique jusqu'à 35 compris et alk est un alkylène $C_2$-$C_5$ linéaire de formule $-(CH_2)n-$, dans laquelle n est un nombre entier de 2 à 5 compris, et leurs sels.

**4.** Procédé selon la revendication 1 de préparation de l'acide 3-(4-phénylpipéridino)-1-hydroxy-propane-1,1-diphosphonique ou d'un de ses sels.

**5.** Procédé selon la revendication 1 de préparation de l'acide 3-(3-phénylpyrrolidino)-1-hydroxy-propane-1,1-diphosphonique ou d'un de ses sels.

**6.** Procédé selon la revendication 1 de préparation de l'acide 3-[4-(p-méthoxyphényl)pipéridino]-1-hydroxy-propane-1,1-diphosphonique ou d'un de ses sels.

**7.** Procédé selon la revendication 1 de préparation de l'acide 3-[4-(p-chlorophényl)pipéridino]-1-hydroxy-propane-1,1-diphosphonique ou d'un de ses sels.

**8.** Procédé selon la revendication 1 de préparation de l'acide 3-[4-(p-fluorophényl)pipéridino]-1-hydroxy-propane-1,1-diphosphonique ou d'un de ses sels.

**9.** Procédé selon la revendication 1 de préparation de l'acide 4-(4-phénylpipéridino)-1-hydroxy-butane-1,1-diphosphonique ou d'un de ses sels.

**10.** Procédé selon la revendication 1 de préparation de l'acide 3-[6-endo-(p-chlorophényl)-3-aza-bicyclo-[$3.1^{1,5}.1^{1,5}$]heptyl-3]-1-hydroxy-propane-1,1-diphosphonique ou d'un de ses sels.

**11.** Procédé selon la revendication 1 de préparation de l'acide 6-(4-phénylpipéridino)-1-hydroxy-hexane-1,1-diphosphonique ou d'un de ses sels.

**12.** Procédé selon la revendication 1 de préparation de l'acide 3-[4-(p-méthylphényl)pipéridino]-1-hydroxy-propane-1,1-diphosphonique ou d'un de ses sels.

**13.** Procédé selon la revendication 1 de préparation de l'acide 3-(4-phényl-octahydro-azocino)-1-hydroxy-propane-1,1-diphosphonique ou d'un de ses sels.

**14.** Procédé selon la revendication 1 de préparation de l'acide 3-[4-(m-fluorophényl)pipéridino]-1-hydroxy-propane-1,1-diphosphonique ou d'un de ses sels.

**15.** Procédé selon la revendication 1 de préparation de l'acide 3-(1-phénylpipérazino)-1-hydroxy-propane-1,1-diphosphonique ou d'un de ses sels.

**16.** Procédé selon la revendication 1 de préparation de l'acide 3-[3-(p-chlorophényl)pyrrolidino]-1-hydroxy-propane-1,1-diphosphonique ou d'un de ses sels.

**17.** Procédé selon la revendication 1 de préparation de l'acide 3-[6-exo-(p-chlorophényl)-3-aza-bicyclo-[$3.1^{1,5}.1^{1,5}$]heptyl-3]-1-hydroxy-propane-1,1-diphosphonique ou un de ses sels.

**18.** Procédé selon la revendication 1 de préparation de l'acide 3-(1-phényl-3-aza-bicyclo[$3.1^{1,5}.1^{1,5}$]-heptyl-3)-1-hydroxy-propane-1,1-diphosphonique ou d'un de ses sels.

**19.** Procédé de production de préparations pharmaceutiques, caractérisé en ce qu'on mélange un composé obtenu selon l'une des revendications 1-18 avec les additifs pharmaceutiques usuels.